# EUROPEAN PATENT SPECIFICATION

(11) **EP 3 207 892 B1**
(45) Date of publication and mention of the grant of the patent: **25.11.2020**
(21) Application number: 17156304.2
(22) Date of filing: 15.02.2017
(51) Int. Cl.: A61B 18/12, A61B 18/14, A61N 1/40

(54) **APPARATUS FOR RADIOFREQUENCY TREATMENTS**
VORRICHTUNG FÜR HOCHFREQUENZBEHANDLUNGEN
APPAREIL DE TRAITEMENT PAR RADIOFRÉQUENCES

(30) Priority: 18.02.2016 IT UB20160858
(43) Date of publication of application: 23.08.2017
(73) Proprietor: GT3 S.r.l., 40124 Bologna (IT); Mazzanti, Roberta, 40050 Loiano (BO) (IT)
(72) Inventor: GRAMOLI, Pietro Tomaso, 40050 Loiano (BO) (IT); LAURITA, Claudio, 40054 Budrio (IT)
(74) Representative: Modiano, Micaela Nadia

(56) References cited:
- WO-A1-2008/153999
- US-A1- 2004 210 214
- US-A1- 2015 025 513

## Description

The present invention relates to an apparatus for radiofrequency treatments.

Aesthetic/medical radiofrequency therapy utilizes the effect generated by adapted medical devices, which determines a noninvasive "reshaping" of tissues, with an action that is particularly effective in contrasting skin relaxation of the face and body.

The principle on which radiofrequency is based is the generation of a thermal shock in the deepest layers of the skin in order to trigger a regenerative response on the part of the body. The application of radiofrequency causes the deep layers of the skin to reach temperatures proximate to or greater than 40°C while keeping the epidermis at lower temperatures so that it remains protected at all times during the treatment.

This entails an immediate distension of collagen fibers, a stimulation of fibroblasts to produce new collagen, a release of cytokines and a regeneration of the matrix, a decrease in the contractility of the facial mimetic muscles, and a reduction in the percentage of peroxide radicals, with a consequent antioxidant topical effect.

The result is a distension of surface wrinkles and a general improvement of the appearance of the skin.

The effect is already visible at the end of the treatment by virtue of the contraction of the collagen fibers "("flash effect").

The deep effect, with production of new collagen, instead occurs several weeks after the beginning of the treatment, since the production of new collagen occurs approximately one month after treatment.

The lifting effect that is obtained is a "gentle" effect, since it is obtained from the physiological response of the body; for this reason it persists for a rather long time and is maximally appreciable during the months that follow the treatment. It has already been written that the electromagnetic radiofrequency field is generated by a high-frequency alternating electric current (above 100 kHz), the flow of which changes direction very rapidly and which does not stimulate the nervous and muscle tissue but has a controlled "thermal effect" due to the increase of the temperature of the dermis.

The biophysical effect of radiofrequency is in fact based on the conversion of electric power into heat: heating occurs as a consequence of the molecular oscillation caused by a rotational displacement of the intracellular electrolytes.

The energy emitted in radiofrequency does not vary if it is used in a monopolar configuration (a single handpiece and a conducting plate which constitutes a second electrode) or a bipolar one (one handpiece with two or more electrodes). Capacitive radiofrequency delivers uniform and constant heat in a predictable manner, being able to reach depths of up to 7 mm, affecting the deep dermis and the "fibrous connective strands" of the subcutaneous region, down to the muscle fascia. The heat generated at the level of the surface dermis and deep dermis and of the adipose tissue causes denaturation of the collagen fibers (5-20%) with consequent immediate contraction of the fibers and progressive effect in the subsequent weeks.

The effects of hyperthermia are neocollagenesis (type 1 collagen) by stimulation of the activity of the fibroblasts, an improving action on skin turgor and tone, an increase in the exchange of substances between the tissues and the blood vessels. All this without thermal damage and with a high safety profile.

The treatment is possible by virtue of a presence of an apparatus that delivers an electric current at radiofrequency (comprised by way of indication between 100 kHz and 2.5/3 MHz), providing it to a respective electrode (in the case of a monopolar handpiece) or to two distinct electrodes (in the bipolar case).

In the case of radiofrequency treatments of the resistive type, the electrode made of conducting material makes direct contact with the patient.

If instead reference is made to radiofrequency treatments of the capacitive type, the electrode is coated with a layer of dielectric (i.e., a material with very low electrical conductivity) and induces an electrical field that varies with the driving frequency (the frequency of the current that reaches the corresponding electrode).

The tissues of the patient that face the electrode (directly in contact with it or with the interposition of the layer made of insulating material) are therefore subjected to an oscillating radiofrequency electromagnetic field which generates the effects described previously thereon.

The thermal effects produced by radiofrequency therapy are also used in the medical field for the treatment of some diseases.

Increasingly often, operators in the field resort to auxiliary devices to detect the temperature of the tissues of the patient that are being subjected to the treatment.

In this manner they can monitor continuously the trend of the temperature of the treated tissues (and of the adjacent ones) in order to optimize the treatment according to the ideal clinical and/or operational parameters provided for it.

This entails the need to use a thermographic detection device, in addition to the radiofrequency apparatus, which can provide the information required for the planning of a correct treatment.

It is not always possible to achieve correct alignment of the thermographic detection device (as a function of the space occupations and of the spaces available in the environment in which the radiofrequency treatment is performed) with the patient (in particular with the corresponding area subjected to the radiofrequency treatment), and therefore the logistics of the work station becomes a fundamental element in order to be able to obtain the best results of the medical and/or aesthetic type.

The quality of the thermographic detection depends on the characteristics of the device being used.

Since radiofrequency treatment leads to better results as the ideal thermal conditions induced in the tissues are better approximated, it is evident that the support offered by the thermographic device is fundamental and that said device must be appropriately coordinated with the radiofrequency apparatus to be assisted. Document US-A-2004/210214 discloses an apparatus according to the preamble of claim 1.

The invention is defined in claim 1. Any embodiment which is in contradiction to the subject-matter of claim 1 is not part of the invention. The aim of the present invention is to solve the problems described above, by proposing an apparatus for radiofrequency treatments that allows to monitor the trend of the temperature in the area of the patient that is subjected to the treatment and in the adjacent ones.

Within this aim, an object of the invention is to propose an apparatus for radiofrequency treatments that ensures the execution of ideal treatment cycles, in accordance with the provided clinical and/or aesthetic standards.

Another object of the invention is to propose an apparatus for radiofrequency treatments that can be installed even in small work stations.

A further object of the present invention is to provide an apparatus for radiofrequency treatments that is low cost, is relatively simple to provide in practice and is safe in application.

This aim and these and other objects which will become better apparent hereinafter are achieved by an apparatus for radiofrequency treatments, of the type comprising at least one cable for connection to a power supply and control unit and at least one handpiece provided with an electrode, characterized in that said unit comprises a thermal camera that is functionally associated with a screen for displaying the acquired data, said thermal camera being orientable toward a region of a patient that is subjected to the radiofrequency treatment of a type chosen preferably between medical and aesthetic.

Further characteristics and advantages of the invention will become better apparent from the description of a preferred but not exclusive embodiment of the apparatus for radiofrequency treatments according to the invention, which is illustrated for the purposes of non-limiting example in the accompanying drawing wherein:

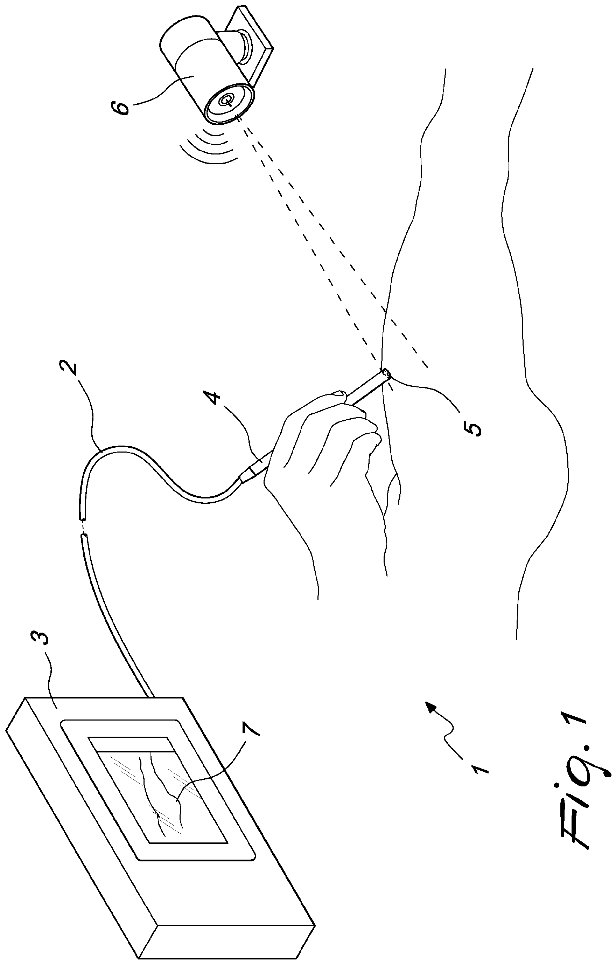

Figure 1 is a schematic perspective view of an apparatus according to the disclosure.

With reference to the figure, an apparatus according to the disclosure, generally designated by the reference numeral 1, comprises at least one cable 2 for connection to a power supply and control unit 3, and at least one handpiece 4 provided with an electrode 5.

Each handpiece 4 can comprise electrodes 5 in a different number and having different dimensions and shape.

In particular, the electrodes 5 can comprise a coating layer made of insulating material (more specifically material having a very low electrical conductivity, which therefore can be defined as an electrical insulator or a dielectric) and have a different shape and dimensions as a function of the type of treatment to which they are dedicated.

According to an alternative constructive solution, the electrodes 5 can be made of conducting material and can have a planar, contoured or needlelike shape (the latter for invasive subcutaneous, infra-muscular and similar treatments).

The power supply and control unit 3 can advantageously comprise, according to the invention, a thermal camera 6 which is functionally associated with a screen 7 for displaying the acquired data.

The pair of components constituted by the thermal camera 6 and by the display screen 7 (integrated in the apparatus 1 according to the invention) in practice behaves like a thermograph.

It should be specified appropriately that the thermal camera 6 can be oriented validly toward the region of the patient that is subjected to the radiofrequency treatment of a type chosen preferably from medical and aesthetic.

The thermal camera 6 can be arranged providing means of an orientable type for coupling to it.

In practice, therefore, with the adoption of the apparatus 1 according to the invention, the operator (generally a physician or a specialized worker) can perform radiofrequency treatments of a particular area of the patient, being able to view the trend of the temperature of the tissues of said area (and of the regions adjacent thereto) directly by viewing the display screen 7 integrated in the apparatus 1.

The treatment can thus be controlled and varied constantly in order to maintain the ideal heating conditions of the treated tissues, which ensure obtainment of the best results (on the therapeutic level and/or on the aesthetic level).

In order to identify with the highest precision a type of handpiece 4 which, combined with the presence of the thermal sensor and of the display screen 7, would ensure the execution of particularly efficient radiofrequency treatments, it is specified that the present invention can be used extensively with apparatuses 1 that use capacitive radiofrequency handpieces 4.

In a version that is particularly safe and of proven effectiveness, in the handpiece 4 the layer of insulating material can be constituted validly by dielectric materials of the type chosen preferably from polymers, ceramic materials, mica, paper, and derivatives thereof.

As an alternative, the adoption of handpieces 4 provided with electrodes 5 having a planar, contoured or needlelike shape, so long as they are made of conducting material (with high electrical conductivity) for radiofrequency treatments of the resistive type, is provided. It is specified that the electrodes 5 for radiofrequency treatments of the resistive type with a needlelike shape allow treatments of the invasive type: the needlelike electrode 5 can in fact be driven into the tissues of the patient in order to affect an internal portion thereof with the treatment.

According to the invention, the power supply and control unit 3 is conveniently accommodated within a substantially box-like body.

The display screen 7 is arranged conveniently on the face of the box-like body that will be directed toward the operator who is using the handpiece 4.

The face for accommodating the screen 7, depending on the type of box-like body that is used, can be the front one, one of the side ones or the upper one, although the possibility to install the screen 7 in the rear face in particular versions is not excluded.

It is deemed necessary to specify that the thermal camera 6 will be functionally associated with the power supply and control unit 3 assigned to the power supply of the electrode 5 of the handpiece 4.

This particular constructive architecture allows the feedback adjustment of the intensity of the radiofrequency emission of the electrode 5. Depending on the thermal detection performed, it might in fact be useful to modulate the emitted wave (in terms of intensity, but optionally also in terms of frequency) in order to prevent certain regions from undergoing excessive overheating, facilitating instead the increase in temperature in other colder regions.

The term "thermography" is understood to reference the two-dimensional visualization of the irradiation measurement. By using a thermal camera 6 (an instrument for performing checks of the thermographic type) it is possible to perform nondestructive and nonintrusive temperature detections (in particular remote detections). Thermal cameras 6 detect radiation in the infrared range of the electromagnetic spectrum and perform measurements which are correlated to the emission of this radiation.

The thermal cameras 6 adopted in the present invention must be able to detect the temperatures of the analyzed bodies by measuring the intensity of the infrared radiation emitted by the body being considered. It should be specified that all bodies, at a temperature higher than absolute zero, emit radiation in the infrared range.

Thermography allows to display absolute values and variations in temperature of the objects regardless of their lighting in the visible range. The quantity of emitted radiation increases proportionally to the fourth power of the absolute temperature of an object.

Starting from the detected radiation, it is therefore possible to obtain temperature maps of the exposed surfaces, which can be used to monitor the effects of the radiofrequency therapy.

The image shown on the display screen 7 is in fact built validly on a matrix of a certain number of pixels by a certain number of lines. A processor controlled by the thermal camera 6 and connected to the display screen 7 therefore detects the value of the energy stored by each individual pixel and generate an image, in black and white or in conventional shades of color, of the observed object.

From a purely application-related viewpoint, it is specified that the display screen 7 can be of the type chosen preferably from an LCD screen, a plasma screen, a cathode-ray tube screen, a touchscreen, a projector and the like.

It is further important to specify that the power supply and control unit 3 can be conveniently connected, even remotely, to optional components, such as a screen, a personal computer, a smartphone, a tablet, a data storage and memory device, and the like.

In this manner it is possible to acquire and store the images acquired by the sensor (thermal camera 6) or have a second display screen which is remotely connected to the power supply and control unit 3.

Such unit 3 might be provided with a transceiver (for example at radiofrequency, Wi-Fi, Bluetooth® and the like) for connection to a remote device.

For example, it might be necessary (or useful) for the operator to have a second screen to allow correct viewing even during treatment steps in which he/she is forced to maintain positions that prevent him/her from viewing the main display screen 7.

In this case, he/she might use a smartphone or a tablet as a remote screen.

The coupling of the apparatus 1 according to the invention to other types of device such as smart glasses (multifunctional technological eyewear) or smart watches (multifunctional technological watches) used by the operator is not excluded.

According to an embodiment of particular interest in practice and in application, the box-like body that contains the power supply and control unit 3, the display screen 7 and the thermal camera 6 and to which the at least one handpiece 4 is connected has a shape and dimensions that are complementary to those of a transport container of the type chosen preferably from a suitcase, a trolley, a trunk and the like.

The advantage of this constructive solution resides in that it is possible to allow the use of said apparatus 1 in multiple surgeries, with a considerable reduction in investments.

The box-like body itself might also be shaped like a trolley in order to facilitate its easy transport from one surgery to another.

Advantageously, the present invention solves the problems described earlier, proposing an apparatus 1 for radiofrequency treatments that allows to monitor the trend of the temperature in the area of the patient that is subjected to the treatment and in the adjacent ones.

Validly, the apparatus for radiofrequency treatments according to the invention ensures the execution of ideal treatment cycles, in accordance with the provided clinical and/or aesthetic standards.

It in fact allows feedback control, based on the detection of the temperature of the region subjected to the treatment, allowing to keep it within the provided ideal ranges.

Feedback control can be performed by the operator, by monitoring continuously the display screen and manually varying the parameters of the radiofrequency emission (intensity and/or frequency).

As an alternative, it is possible to provide automatic feedback control, which minimizes the risk that the operator might make mistakes, subjecting areas of the patient that are already overheated to any additional wave trains emitted by the handpiece.

Usefully, the apparatus according to the invention can also be installed in small work stations; the very fact that the display screen 7 is integrated in the box-like body in fact allows to use the apparatus 1 according to the invention in any work environment that allows to accommodate said box-like body.

Conveniently, the apparatus 1 for radiofrequency treatments according to the invention is relatively simple to provide in practice, as well as modest in cost (if compared with the only currently known possibility, which provides for the execution of controlled radiofrequency treatments, in which it is necessary to use a radiofrequency apparatus and a thermograph which are separate).

These characteristics make the apparatus according to the invention an innovative solution with assured practical application.

The invention thus conceived is susceptible of numerous modifications and variations, all of which are within the scope of the appended claims; all the details may further be replaced with other technically equivalent elements.

In particular, the thermal camera 6 provided according to the invention can be coupled to a respective supporting element to arrange it proximate to the area of the patient to be monitored during the radiofrequency treatment.

In the exemplary embodiments shown, individual characteristics, given in relation to specific examples, may actually be interchanged with other different characteristics that exist in other exemplary embodiments.

In practice, the materials used, as well as the dimensions, may be any according to the requirements and the state of the art.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus (1) for radiofrequency treatments, the apparatus comprising a power supply and control unit (3), at least one handpiece (4) provided with an electrode (5) being adapted to emit radiofrequency energy, and at least one cable (2) for connection of the handpiece to the power supply and control unit (3), said unit (3) comprising a thermal camera (6) that is functionally associated with a screen (7) for displaying the acquired data, said thermal camera (6) being orientable toward a region of a patient that is subjected to the radiofrequency treatment of a type chosen preferably between medical and aesthetic, the screen being comprised by the apparatus, **characterized in that** said power supply and control unit (3) is accommodated within a substantially box-like body, said display screen (7) being arranged on the face of said box-like body that is directed toward the operator who is using said handpiece (4), wherein the box-like body is comprised by the apparatus.

2. The apparatus according to claim 1, **characterized in that** said electrode (5) is coated with a layer of insulating material, of the type chosen preferably from polymers, ceramic materials, paper, mica, and derivatives thereof.

3. The apparatus according to claim 1, **characterized in that** said electrode (5) is constituted by an element with high electrical conductivity, of the type chosen preferably from metals and carbon derivatives, and has a shape that is preferably chosen from planar, contoured and needlelike.

4. The apparatus according to claim 1, **characterized in that** said thermal camera (6) is functionally associated with said power supply and control unit (3) preset for the power supply of said electrode (5), in order to provide the feedback adjustment of the intensity of the radiofrequency emission of said electrode (5).

5. The apparatus according to claim 1, **characterized in that** said display screen (7) is of the type chosen preferably from an LCD screen, a plasma screen, a cathode ray tube screen, a touchscreen, a projector, and the like.

6. The apparatus according to claim 1, **characterized in that** said power supply and control unit (3) is connected, even remotely, to optional components, such as a screen, a personal computer, a smartphone, a tablet, a memory and data storage device, and the like.

7. The apparatus according to claim 1, **characterized in that** said box-like body has a shape and dimensions that are complementary to those of a transport container of the type chosen preferably from a suitcase, a trolley, a trunk, and the like.

8. The apparatus according to claim 1, **characterized in that** said thermal camera (6) is connected to said apparatus (1) in a mode that is chosen preferably between wired and remote.

## Patentansprüche

1. Eine Vorrichtung (1) für Hochfrequenzbehandlungen, wobei die Vorrichtung eine Stromversorgungs- und Steuereinheit (3), mindestens einen Griff (4), der mit einer Elektrode (5) ausgestattet ist, welche ausgebildet ist, um Hochfrequenzenergie auszusenden, und mindestens ein Kabel (2) zur Verbindung des Griffs mit der Stromversorgungs- und Steuereinheit (3) umfasst,
wobei die Einheit (3) eine Wärmekamera (6) umfasst, die funktional mit einem Bildschirm (7) für die Anzeige der erfassten Daten verbunden ist, wobei die Wärmekamera (6) auf einen Bereich eines Patienten ausgerichtet werden kann, welcher der Hochfrequenzbehandlung von einer Art unterzogen wird, die vorzugsweise gewählt ist aus medizinisch und ästhetisch; wobei der Bildschirm von der Vorrichtung umfasst ist,
**dadurch gekennzeichnet, dass** die Stromversorgungs- und Steuereinheit (3) in einem im Wesentlichen kastenähnlichen Körper untergebracht ist, wobei der Bildschirm (7) auf der Seite des kastenähnlichen Körpers angeordnet ist, die dem Bediener, der den Griff (4) benutzt, zugewandt ist,
wobei der kastenähnliche Körper von der Vorrichtung umfasst ist.

2. Die Vorrichtung gemäß Anspruch 1, **dadurch gekennzeichnet, dass** die Elektrode (5) mit einer Schicht aus Isoliermaterial von der Art beschichtet ist, die vorzugsweise gewählt ist aus Polymeren, Keramikmaterialien, Papier, Glimmer und Derivaten davon.

3. Die Vorrichtung gemäß Anspruch 1, **dadurch gekennzeichnet, dass** die Elektrode (5) aus einem Element mit hoher elektrischer Leitfähigkeit, von der Art, die vorzugsweise gewählt ist aus Metallen und Kohlenstoffderivaten, besteht und eine Form hat, die vorzugsweise gewählt ist aus planar, konturiert und nadeiförmig.

4. Die Vorrichtung gemäß Anspruch 1, **dadurch gekennzeichnet, dass** die Wärmekamera (6) funktional mit der Stromversorgungs- und Steuereinheit (3) verbunden ist, die für die Stromversorgung der Elektrode (5) voreingestellt ist, um für die Rückkopplungsanpassung der Intensität der Hochfrequenzemission der Elektrode (5) zu sorgen.

5. Die Vorrichtung gemäß Anspruch 1, **dadurch gekennzeichnet, dass** der Bildschirm (7) von der Art ist, die vorzugsweise gewählt ist aus einem LCD-Bildschirm, einem Plasmabildschirm, einem Kathodenstrahlröhren-Bildschirm, einem Touchscreen, einem Projektor und dergleichen.

6. Die Vorrichtung gemäß Anspruch 1, **dadurch gekennzeichnet, dass** die Stromversorgungs- und Steuereinheit (3), auch über Fernverbindung, mit fakultativen Komponenten wie einem Bildschirm, einem PC, einem Smartphone, einem Tablet, einer Speicher- und Datenspeichervorrichtung und dergleichen verbunden ist.

7. Die Vorrichtung gemäß Anspruch 1, **dadurch gekennzeichnet, dass** der kastenähnliche Körper eine Form und Maße hat, die komplementär zu denjenigen eines Transportbehälters von der Art sind, die vorzugsweise gewählt ist aus einem Koffer, einem Rollwagen, einem Kofferraum und dergleichen.

8. Die Vorrichtung gemäß Anspruch 1, **dadurch gekennzeichnet, dass** die Wärmekamera (6) mit der Vorrichtung (1) auf eine Art verbunden ist, die vorzugsweise aus Kabelverbindung und Fernverbindung gewählt ist.

## Revendications

1. Appareil (1) destiné à des traitements par radiofréquences, l'appareil comportant une unité d'alimentation électrique et de commande (3), au moins une pièce à main (4) pourvue d'une électrode (5) étant adaptée pour émettre de l'énergie radiofréquence, et au moins un câble (2) pour le raccordement de la pièce à main à l'unité d'alimentation électrique et de commande (3), ladite unité (3) comportant une caméra thermique (6) qui est fonctionnellement associée à un écran (7) pour afficher les données acquises, ladite caméra thermique (6) pouvant être orientée vers une région d'un patient qui est soumis au traitement par radiofréquences d'un type de préférence choisi entre médical et esthétique, l'écran étant compris dans l'appareil,
**caractérisé en ce que** ladite unité d'alimentation électrique et de commande (3) est reçue à l'intérieur d'un corps sensiblement analogue à un boîtier, ledit écran d'affichage (7) étant agencé sur la face dudit corps analogue à un boîtier qui est dirigée vers l'opérateur qui utilise ladite pièce à main (4), dans lequel le corps analogue à un boîtier est compris dans l'appareil.

2. Appareil selon la revendication 1, **caractérisé en ce que** ladite électrode (5) est revêtue d'une couche de matériau isolant, du type de préférence choisi parmi des polymères, des céramiques, du papier, du mica et des dérivés de ceux-ci.

3. Appareil selon la revendication 1, **caractérisé en ce que** ladite électrode (5) est constituée d'un élément ayant une conductivité électrique élevée, du type de préférence choisi parmi des métaux et des dérivés du carbone, et a une forme qui est de préférence choisie parmi plane, galbée et aciculaire.

4. Appareil selon la revendication 1, **caractérisé en ce que** ladite caméra thermique (6) est fonctionnellement associée à ladite unité d'alimentation électrique et de commande (3) préréglée pour l'alimentation électrique de ladite électrode (5) afin d'assurer le réglage d'asservissement de l'intensité de l'émission radiofréquence de ladite électrode (5).

5. Appareil selon la revendication 1, **caractérisé en ce que** ledit écran d'affichage (7) est du type de préférence choisi parmi un écran à cristaux liquides (LCD), un écran à plasma, un écran à tube cathodique, un écran tactile, un projecteur et analogue.

6. Appareil selon la revendication 1, **caractérisé en ce que** ladite unité d'alimentation électrique et de commande (3) est connectée, même à distance, à des composants optionnels, tels qu'un écran, un ordinateur personnel, un téléphone intelligent, une tablette, un dispositif de mémoire et de stockage de données, et analogue.

7. Appareil selon la revendication 1, **caractérisé en ce que** ledit corps analogue à un boîtier a une forme et des dimensions qui sont complémentaires de celles d'un conteneur de transport du type de préférence choisi parmi une valise, un chariot, une mallette et analogue.

8. Appareil selon la revendication 1, **caractérisé en ce que** ladite caméra thermique (6) est connectée audit appareil (1) dans un mode qui est de préférence choisi entre filaire et distant.
